(19) Europäisches Patentamt
European Patent Office
Office européen des brevets

(11) Publication number: **0 188 053**
**A2**

(12) # EUROPEAN PATENT APPLICATION

(21) Application number: 85305900.4

(22) Date of filing: 19.08.85

(51) Int. Cl.[4]: C 12 N 9/02

(30) Priority: 17.01.85 CA 472274

(43) Date of publication of application: 23.07.86 Bulletin 86/30

(84) Designated Contracting States: AT BE CH DE FR GB IT LI LU NL SE

(71) Applicant: Friesen, Albert D.
6 Rice Road
Winnipeg Manitoba, R3T 3N4(CA)

(71) Applicant: Weselake, Randall J.
27 Greenmount Road
Winnipeg Manitoba, R2J 1T3(CA)

(72) Inventor: Friesen, Albert D.
6 Rice Road
Winnipeg Manitoba, R3T 3N4(CA)

(72) Inventor: Weselake, Randall J.
27 Greenmount Road
Winnipeg Manitoba, R2J 1T3(CA)

(74) Representative: Lightfoot, Robert Oscar et al,
Raworth, Moss & Cook 36 Sydenham Road
Croydon Surrey, CR0 2EF(GB)

(54) **Purification of superoxide dismutase.**

(57) A process for purifying the enzyme, superoxide dismutase, by passing an aqueous solution containing the enzyme, which is free from hemoglobin, through a metal chelating affinity chromatographic column, for example an immobilized copper ion column, and eluting an enzyme solution having increased specific activity.

EP 0 188 053 A2

Croydon Printing Company Ltd.

Purification of Superoxide Dismutase

This invention relates to a process for the purification and production of the enzyme commonly referred to as superoxide dismutase (SOD). More particularly, the invention relates to a process for the purification and production of superoxide dismutase by use of a metal chelating chromatographic procedure. The invention is particularly suitable for use in the purification of superoxide dismutase from human outdated red blood cells, or the purification of recombinant human superoxide dismutase obtained from bacteria capable of synthesizing this enzyme.

The invention as claimed herein is a process for purifying superoxide dismutase which comprises subjecting an aqueous solution containing superoxide dismutase which is free from hemoglobin to a metal chelating affinity chromatographic procedure and recovering an aqueous solution containing superoxide dismutase having increased specific activity of superoxide dismutase.

The aqueous solution containing superoxide dismutase, which is free from hemoglobin, to be used as starting material, may be conveniently obtained, if desired, from outdated human red blood cells. Such cells, preserved in a suitable medium, such as a citrate-phosphate-dextrose-adenine medium, may be lysed by means of deionized water. The lysate so obtained may be freed from hemoglobin in a variety of ways, such as a) passage through an anion exchange chromatographic column using, for example, a

cross-linked agarose derivative such as diethylaminoethyl (DEAE) - *Sepharose, or b) a precipitation technique or c) an affinity technique. The starting material for the metal chelating affinity chromatographic procedure may alternatively be an extract of bacterial cells which are capable of synthesizing human SOD.

It is preferred to use an anion exchange chromatographic column for the preliminary purification procedure to remove hemoglobin. Any suitable anion exchanger may be used, such as a cross-linked agarose derivative, for example DEAE - *Sepharose, in conjunction with an appropriate equilibration buffer which may be for example potassium phosphate at pH 6.4. The conditions for separating the superoxide dismutase from the hemoglobin on the anion exchange column by elution may be varied considerably. A buffer with a molar concentration of 0.1M or higher and with a pH of about 5 or higher is a suitable buffer for elution of the superoxide dismutase. Such buffers may be, for example, 0.1M potassium phosphate solution used alone or 10mM potassium phosphate containing either 0.1M sodium chloride or 0.1M potassium chloride. Operation of the column at a temperature of about 4°C and at a flow rate of about 150 ml/hour provides an eluate in the form of an aqueous solution containing superoxide dismutase which is suitable for use in the subsequent metal chelating affinity chromatographic procedure.

As an alternative method for providing the starting material containing superoxide dismutase, it is possible to subject red blood cells to a saline wash followed by a tangential flow ultrafiltration system and rupture of the cells hypotonically before the lysate is applied to the anion exchange column. Thus, the red blood cells may be washed with about 10 volumes of saline using a tangential flow ultrafiltration system. The saline wash is carried out twice followed by concentration of red blood cells

using the same ultrafiltration system. Cells are then ruptured, hypotonically, by the addition of 3 volumes of distilled water. The cell membranes are removed from the soluble proteins using the ultrafiltration apparatus and filtered lysate is then applied to the anion exchange column. The ultrafiltration process removes the bulk of the plasma proteins present in red blood cell concentrate and thus increases the efficiency of the process to provide the starting material in the form of an aqueous solution containing superoxide dismutase. Another variation of the ultrafiltration process that can be used is the removal of insoluble material from red blood cell lysates by filtration without prior washing of the cells with saline.

As indicated above, the starting solution to be used in the metal chelating affinity chromatographic procedure may be an extract of bacterial cells capable of synthesizing human superoxide dismutase. Such an extract would be suitably prepared according to preliminary purification procedures before being applied to the chromatographic column.

The metal chelating affinity chromatographic procedure may be carried out on a suitable column wherein the chelated metal is attached to an insoluble matrix. A suitable matrix may be a cross-linked agarose derivative, a derivative from vinyl polymers or a derivative from cellulose or silica or polyacrylamides. Suitable cross-linked agarose derivatives may be, for example, inimodiacetate-*Sepharose 6B or ethylene-diamine-*Sepharose or tris-(carboxymethyl) ethylenediamine-*Sepharose. Of these, it is preferred to use iminodiacetate-*Sepharose 6B as the column of choice. A suitable matrix as a derivative from vinyl polymers may be iminodiacetate-*Fractogel. Other matrix columns may be prepared from an iminodiacetate-linked derivative of cellulose,

polyacrylamide, silica or synthetic polymeric supports. A desirable matrix is iminodiacetate cellulose.

It is to be understood that the commercially available product known as iminodiacetate-Sepharose* 6B is a beaded agarose gel consisting of the chelating agent, iminodiacetic acid, coupled to agarose after epoxy activation, the latter introducing some cross-linking into the matrix. The product thus has bis-(carboxymethyl)-amino groups coupled to agarose via long, stable, hydrophilic spacer arms as shown by the formula:

$$agarose\text{-}O\text{-}CH_2CHOHCH_2O(CH_2)_4OCH_2CHOHCH_2N(CH_2COOH)_2$$

The metal to be immobilized on the column is preferably copper and the immobilization procedure may be carried out by applying an aqueous solution of a copper salt to the column.

A variety of copper salts may be used, such as copper sulphate, copper chloride and copper nitrate. A preferred copper salt is copper sulphate and a 50mM aqueous solution of copper sulphate may be applied to a column of iminodiacetate-Sepharose* 6B to activate the column. A buffer having a pH from about 6 to about 8 provides suitable conditions for binding the superoxide dismutase to the copper chelating column. When the metal chelating affinity column has been equilibrated, the aqueous solution containing superoxide dismutase, prepared as starting material, may be applied to the immobilized copper column. Subsequent elution provides an aqueous solution containing highly purified superoxide dismutase showing a specific activity of about 3500 unit/mg. As a suitable eluant, there may be used 20mM sodium citrate buffer at pH 5.0 or 20mM sodium citrate buffer at pH 5.0 containing 1M sodium chloride.

It will be appreciated by those skilled in the art that other suitable columns and other copper salts, with appropriate buffers, may be used to provide the immobilized copper chelating affinity column on which the aqueous solution of superoxide dismutase might be applied and thereafter eluted to provide highly purified superoxide dismutase.

The process of this invention is particularly valuable when the copper chelating column is prepared from an aqueous solution of copper sulphate applied to a column of iminodiacetate-Sepharose* 6B. The process is further enhanced when the starting material to be used on such copper chelating affinity column is an aqueous solution containing superoxide dismutase which has been obtained as an eluate from a chromatographic column containing diethylaminoethyl (DEAE) - Sepharose* to remove hemoglobin from human red blood cell lysate.

Thus, as a further feature of the invention, as claimed herein there is provided a process for purifying superoxide dismutase which comprises subjecting human red blood cell lysate to an anion exchange chromatographic procedure, wherein the anion exchanger is DEAE - Sepharose*, to provide an aqueous solution containing superoxide dismutase free from hemoglobin and then subjecting the aqueous solution to an immobilized copper ion chromatographic procedure, wherein the immobilized copper ion is prepared from an aqueous solution of a copper salt, such as copper sulphate, copper chloride or copper nitrate on a column of iminodiacetate-Sepharose* 6B, and then eluting the column to provide an aqueous solution containing superoxide dismutase having increased specific activity of superoxide dismutase.

The invention is illustrated by, but not limited by, the following Examples.

## EXAMPLE I

The following example describes a procedure for purifying superoxide dismutase (SOD) by chromatography on a column of immobilized copper ions.

Outdated red blood cells in suspension (15 ml), preserved in citrate-phosphate-dextrose-adenine, were lysed with an equal volume of deionized water. The lysate so obtained was then applied to a 5 cm x 4 cm column of DEAE - Sepharose* equilibrated with 10mM potassium phosphate buffer (pH 6.4) at $4^{\circ}C$ and at a flow rate of 150 ml/hour. The same temperature and flow rate were maintained throughout the remainder of the chromatographic procedure. After the lysate had been applied, the column was washed with approximately 250 ml of equilibration buffer. Partially purified SOD was then eluted from the DEAE - Sepharose* column with the same equilibration buffer containing 0.1M sodium chloride thereby providing an aqueous solution of partially purified SOD.

A column (5 cm x 2 cm) of iminodiacetate-Sepharose* 6B was activated with 50mM aqueous copper sulphate solution to approximately 3/4 of the bed volume to give an immobilized copper ion column. Prior to application of the partially purified SOD solution, the immobilized copper ion column was equilibrated with 10mM potassium phosphate buffer (pH 6.4) containing 0.1M NaCl. The aqueous solution of partially purified SOD, eluted from the DEAE - Sepharose* column, was applied to the immobilized copper ion column and thereafter washed through with 10mM potassium phosphate buffer containing 1M NaCl. After the effluent reached an absorbance of zero, at 280 nm, the column was washed with 50 ml of 10mM potassium phosphate buffer (pH 6.4). Purified SOD was eluted from the copper ion chelate column with 20mM sodium citrate buffer (pH 5.0). Enzyme activity (Hyland _et al_., Anal. Biochem. 1983, _135_, 280)

and protein determinations (Lowry et al., J. Biol. Chem. 1951, 193, 265) indicated that the specific activity of the SOD increased about 270-fold as a result of this chromatographic procedure on copper ion iminodiacetate-Sepharose* 6B. Recovery of enzyme activity, following this procedure, ranged from 75% to 80% of the total enzyme activity applied to the column.

## EXAMPLE II

Outdated red blood cells (250 ml) were lysed with 500 ml of distilled water. Insoluble material was removed by tangential flow ultrafiltration using a Minitan* ultrafiltration unit equipped with four 0.45 micron cutoff membranes. Approximately 600 ml of filtrate were collected and applied to a 5 cm x 16 cm DEAE - Sepharose* anion-exchange column equilibrated as described in Example I. Following sample application, the anion-exchange column was washed with 3 litres of equilibration buffer. Partially purified SOD was eluted from the same DEAE - Sepharose* column with equilibration buffer containing 0.10M NaCl thereby providing an aqueous solution of partially purified SOD.

A column (5 cm x 6 cm) of iminodiacetate-Sepharose* 6B was activated with 50mM aqueous copper chloride solution or 50mM aqueous copper nitrate solution to approximately 3/4 of the bed volume to give an immobilized copper ion column. This column was then used to purify SOD from DEAE - Sepharose* as described in Example I.

In this case, however, SOD was eluted from the metal chelation affinity column with 20mM sodium citrate buffer at pH 5.0 containing 1M NaCl. Recovery of enzyme activity, following this procedure was about 90% of the total enzyme activity applied to the column.

EXAMPLE III

Outdated red blood cells (50 ml) were washed twice with saline solution. The centrifugation pellet from the second saline wash was lysed with 100 ml of distilled water. Hemoglobin was removed from the lysate by chloroform - ethanol treatment and by treatment with solid potassium phosphate (McCord and Fridovich, J. Biol, Chem. 1969, 244, 6049). The resulting ethanolic phase, containing partially purified SOD, was dialysed overnight against 10mM potassium phosphate buffer at pH 6.4 containing 0.1M NaCl. After dialysis the aqueous solution of partially purified SOD was centrifuged and the supernatant subjected to copper chelation affinity chromatography as described in Example I.

EXAMPLE IV

Outdated red blood cells (25 ml) were lysed with an equal volume of distilled water. Most of the hemoglobin was removed from the lysate by heat treatment at 70°C-80°C for 15 minutes (Gartner et al., Biochem, J. 1984, 221, 549). Following centrifugation to remove insoluble material, the supernatant contained partially purified SOD which was dialyzed against 10mM potassium phosphate buffer at pH 6.4 containing 1M NaCl and further purified by the process of copper chelation affinity chromatography described in Example I.

EXAMPLE V

A column (2 cm x 2 cm) of iminodiacetate *Fractogel was activated with 50mM aqueous copper sulphate solution to approximately 3/4 of the bed volume to give an immobilized copper ion column. The column was equilibrated at pH 6.4 as described in Example I. Ten ml of SOD solution, from DEAE - *Sepharose chromatography, was applied to the

immobilized copper ion column followed by 20 ml of equilibration buffer. SOD was eluted from the metal chelation column with 20mM sodium citrate at pH 5 containing 1M NaCl. Enzyme activity was recovered, completely, following this copper chelation affinity chromatography.

EXAMPLE VI

A column (5 cm x 2 cm) of iminodiacetate *Cellulose was activated with 50mM aqueous copper sulphate solution to approximately 3/4 of the bed volume to give an immobilized copper ion column. The column was equilibrated at pH 6.4 as described in Example I. One hundred ml of SOD solution, from DEAE *Sepharose chromatography, was applied to the immobilized copper ion column followed by extensive washing with equilibration buffer. Purified SOD was eluted from the immobilized copper ion column with 20mM sodium citrate buffer, pH 5.

* Trade Mark

WE CLAIM:

1. A process for purifying superoxide dismutase which comprises subjecting an aqueous solution containing said superoxide dismutase which is free from hemoglobin to a metal chelating affinity chromatographic treatment and recovering an aqueous solution containing superoxide dismutase having increased specific activity of superoxide dismutase.

2. The process of claim 1 wherein the metal chelating affinity chromatographic treatment is a copper chelating affinity chromatographic treatment.

3. The process of claim 1 wherein the metal chelating affinity chromatographic treatment is carried out by use of an immobilized copper ion chromatographic column.

4. The process of claim 1, 2 or 3 wherein the chromatographic treatment uses a matrix which is selected from a cross-linked agarose derivative, a vinyl polymer derivative and cellulose or silica or polyacrylamide derivatives.

5. The process of claim 1, 2 or 3 wherein the chromatographic treatment uses a matrix which is selected from iminodiacetate-Sepharose 6B, ethylenediamine-Sepharose, tris-(carboxymethyl) ethylenediamine-Sepharose, iminodiacetate-Fractogel, iminodiacetate-Cellulose and iminodiacetate-derivatives of silica or polyacrylamide or synthetic polymeric supports.

6. The process of claim 3 wherein the immobilized copper ion chromatographic column is prepared from a column of iminodiacetate-Sepharose 6B or iminodiacetate-Cellulose activated with an aqueous solution of a copper salt.

7. The process of claim 6 wherein the copper salt is copper sulphate, copper chloride or copper nitrate.

8. The process of claim 7 wherein the copper salt is used in the form of a 50mM aqueous copper sulphate, copper chloride or copper nitrate solution.

9. The process of claim 6, 7 or 8 wherein the column of iminodiacetate-Sepharose 6B or iminodiacetate-Cellulose is activated with the aqueous copper salt solution to approximately 3/4 of the bed volume.

10. The process of any one of claims 3 to 9 wherein the immobilized copper ion chromatographic column is equilibrated with a buffer before application of the aqueous solution containing the superoxide dismutase.

11. The process of claim 10 wherein the buffer has a pH of from about 6 to about 8.

12. The process of claim 11 wherein the buffer comprises potassium phosphate or sodium phosphate.

13. The process of claim 12 wherein the buffer is an aqueous 10mM potassium phosphate buffer having a pH of 6.4 and containing 0.1M sodium chloride.

14. The process of any preceding claim wherein the aqueous solution containing superoxide dismutase used as starting material is obtained from human red blood cells.

15. The process of claim 14 wherein the starting material is a human red blood cell lysate.

16. The process of any one of claims 1 to 13 wherein the aqueous solution containing superoxide dismutase used as starting material is obtained from the purification of

recombinant human superoxide dismutase from bacteria capable of synthesizing the enzyme.

17. The process of any one of the preceding claims wherein the aqueous solution containing superoxide dismutase used as starting material is subjected to a chromatographic treatment using an anion exchanger before the metal chelating affinity chromatographic treatment.

18. The process of claim 17 wherein the anion exchanger chromatographic treatment is carried out in an anion exchange column.

19. The process of claim 18 wherein the anion exchange column contains DEAE - Sepharose as the ion exchanger.